# EUROPEAN PATENT SPECIFICATION

(11) **EP 1 146 864 B1**
(45) Date of publication and mention of the grant of the patent: **04.03.2009**
(21) Application number: 00904586.5
(22) Date of filing: 26.01.2000
(51) Int. Cl.: A61K 9/20

(54) **PH INDEPENDENT EXTENDED RELEASE PHARMACEUTICAL FORMULATION**
PH-UNABHÄNGIGE PHARMAZEUTISCHE FORMULIERUNGEN MIT VERLÄNGERTER FREISETZUNG
PREPARATIONS PHARMACEUTIQUES A LIBERATION PROLONGEE INDEPENDANTE DU PH

(30) Priority: 04.02.1999 US 244678
(43) Date of publication of application: 24.10.2001
(73) Proprietor: ABBOTT LABORATORIES, Abbott Park IL 60064-3500 (US)
(72) Inventor: ENGH, Kevin, R., Kenosha, WI 53143 (US); QIU, Yihong, Gurnee, IL 60031 (US); RAO, Venkatramana, Lawrence, KS 66046 (US)
(74) Representative: Modiano, Micaela Nadia
(86) International application number: PCT/US2000/001954
(87) International publication number: WO 2000/045793

(56) References cited:
- EP-A- 0 334 167
- WO-A-97/06787
- US-A- 5 017 613
- US-A- 5 019 398

## Description

### Technical Field

This invention relates to pharmaceutical formulations. More particularly, the present invention concerns extended release pharmaceutical formulations containing an acidic pharmacologic agent which formulations have reduced dependence of release rate on pH.

### Background of the Invention

The normal pH of gastric juices is about pH 1, while the pH in the intestinal tract averages about pH 7. This fact has been used to advantage for years in so-called "enteric coated" pharmaceutical formulations. These formulations are generally in the form of tablets coated with a substance which is insoluble or sparingly soluble in acidic solutions, but which dissolves rapidly at higher pH. Such enteric coated formulations permit the oral administration of drugs which would present problems if released in the stomach, such as irritation of the stomach lining. Moreover, enteric-coated tablets also permit extending the release of a drug over time. For example, a tablet can be formulated by compressing granules containing the drug, some of which granules are enteric coated and some of which are not. As the tablet disintegrates, the non-enteric coated granules dissolve in the stomach, immediately releasing the drug, while the enteric coated granules pass to the intestine before dissolving to release the drug. In this way, release of the drug can be extended over the time the drug is resident in both the stomach and intestine. Such an extended release system is crude, essentially releasing the drug in a bi-modal manner. It is generally desirable to release a drug more smoothly over time than can be done by a partially enteric coated formulation of the type just described.

In the effort to achieve smooth, controllable release of acidic pharmacologic agents, several systems have been devised. These fall into one of three general classes: osmotic systems, dissolution systems, and diffusion systems. An example of an osmotic system is a tablet consisting of a core of drug surrounded by a semi-permeable membrane containing a small orifice. When the tablet is exposed to an aqueous body fluid, water flows into the tablet through the semi-permeable membrane due to the osmotic pressure difference. The drug is then pumped out of the tablet through the orifice at a constant rate controlled by the parameters of drug concentration, orifice diameter, osmotic pressure difference, etc., until the drug concentration inside the tablet falls below saturation.

Dissolution systems take advantage of the inherent dissolution rate of the drug itself, or of a particular salt or derivative. Alternatively, the drug can be coated with a slowly dissolving coating, or by incorporating the drug into a slowly dissolving carrier.

Diffusion systems include both reservoir devices, in which a core of drug is surrounded by a polymeric membrane, and matrix devices in which dissolved or dispersed drug is distributed uniformly throughout an inert polymer matrix. The release of drug from a reservoir system involves the flow of drug through the membrane, and is controlled by Fick's first law of diffusion. Depending upon the shape of the tablet, the equation describing the release will vary.

In matrix systems, the mechanism of drug release is assumed to involve dissolution of the drug from the surface layer of the device first, followed by dissolution from the underlying layer and diffusion through the overlying drug-depleted layer, etc.

The design of a sustained or extended release formulation for drugs which are acidic present particular problems for the pharmaceutical formulator. The solubility of such drugs in gastric juices is typically low as a result of the repression of ionization of the acid by the low pH in the stomach. On the other hand, such acidic drugs dissolve rapidly in the intestine, sometimes more rapidly than desired. The various systems described above lend themselves readily to the formulation of extended release formulations of drugs which are unaffected by pH as they traverse the alimentary canal, but do not provide adequate formulations where the drug has widely varying pH-dependent release rates between the stomach and intestinal tract.

One such acidic pharmacologic agent is 2-propylpentanoic acid, more commonly known as valproic acid (VPA), which is effective in the treatment of epileptic seizures or as an antipsychotic agent. United States Patent 4,988,731 to Meade discloses an oligomer having a 1:1 molar ratio of sodium valproate and valproic acid containing 4 units, and United States Patent 5,212,326 to Meade discloses a stable, non-hygroscopic solid form of valproic acid which comprises an oligomer having 1:1 molar ratio of sodium valproate and valproic acid and containing four to six units. Divalproex sodium (sodium hydrogen divalproate), a comple formed between one mole of 2-propylpentanoic acid and its sodium salt, is one of the most widely accepted antiepileptic agents currently available.

However, despite its efficacy in the treatment of epilepsy, valproic acid is poorly soluble in the stomach and has also been shown to exhibit an elimination half-life which is shorter than other commonly used anti-epileptic agents. Half-lives for the drug of between six and seventeen hours in adults and between four and fourteen hours in children have been reported. This leads to substantial fluctuations in the plasma concentration of the drug, especially in chronic administration. To maintain reasonable stable plasma concentrations, it is necessary to resort to frequent dosing, and the resulting inconvenience to the patient often results in lowered compliance with the prescribed dosing regimen. Moreover, widely fluctuating plasma concentrations of the drug may result in administration of less than therapeutic amounts of the drug in a conservative dosing regimen, or to amounts too large for the particular patient in an aggressive dosing regimen.

To overcome these disadvantages, a concerted effort has been devoted to the discovery of formulations which will maintain more constant plasma levels of acidic drugs in general, and valproic acid in particular, following administration. The ultimate goal of these studies has been the discovery of a formulation which affords stable plasma levels in a once-a-day dosing regimens for such drugs. These efforts fall generally into one of two categories: (a) finding a form of the active ingredient which is more uniformly released to the body metabolically, and (b) finding a formulation which delivers the drug by either a timed- or controlled-release mechanism.

With regard to valproic acid, United States Patent 4,369,172 to Schor, et al. describes, for example, a prolonged release therapeutic composition based on mixtures of hydroxypropylmethyl cellulose, ethyl cellulose and/or sodium carboxymethyl cellulose. The patentees provide a long list of therapeutic agents which they suggest can be incorporated into the formulation including sodium valproate.

United States Patent 4,913,906 to Friedman, et al. discloses a controlled release dosage form of valproic acid, its amide, or one of its salts or esters in combination with a natural or synthetic polymer, pressed into a tablet under high pressure.

United States Patent 5,009,897 to Brinker, et al. discloses granules, suitable for pressing into tablets, the granules comprising a core of divalproex sodium and a coating of a mixture of a polymer and microcrystalline cellulose.

United States Patent 5,019,398 to Daste discloses a sustained-release tablet of divalproex sodium in a matrix of hydroxypropylmethyl cellulose and hydrated silica.

United States Patent 5,055,306 to Barry, et al. discloses an effervescent or water-dispersible granular sustained release formulation suitable for use with a variety of therapeutic agents. The granules comprise a core comprising the active ingredient and at least one excipient, and a water insoluble, water-swellable coating comprising a copolymer of ethyl acrylate and methyl methacrylate and a water soluble hydroxylated cellulose derivative. The patentees suggest a list of therapeutic agents which may be used in the formulation of the invention, including sodium valproate.

United States Patent 5,169,642 to Brinker, et al. disclose a sustained release dosage form comprising granules of divalproex sodium or amides or esters of valproic acid coated with a sustained release composition comprising ethyl cellulose or a methacrylic methyl ester, a plasticizer, a detackifying agent, and a slow-release polymeric viscosity agent.

United States Patent 5,185,159 to Aubert, et al. disclose a formulation of valproic acid and sodium valproate which is prepared without the use of either a binder or a granulating solvent. The formulation optionally contains precipitated silica as an antisticking or detackifying agent.

United States Patent 5,589,191 to Exigua, et al. discloses a slow release sodium valproate tablet formulation in which the tablets are coated with ethyl cellulose containing silicic acid anhydride.

Published PCT application WO 94/27587 to Ayer, *et al*. discloses a method for control of epilepsy by delivering a therapeutic composition of valproic acid or a derivative in combination with a polyalkylene oxide.

Bailer, et al., "Metabolism of Antiepileptic Drugs," pp. 143-151, R. H. Levy, Ed., Raven Press, New York, 1984; Int. J. Pharmaceutics, 20: 53-63 (1984); and Biopharmaceutics and Drug Disposition, 6: 401-411 (1985); and Israel J. Med. Sci., 20: 46-49 (1995) report the pharmacokinetic evaluation of several sustained release formulations, of valproic acid. WO9706787 discloses a method of making a solid interpolymer complex which may be used as a controlled release matrix for a controlled release product for oral administration.

Despite these efforts, however, there remains a need for formulations of acidic pharmacologic agents (in general), and valproic acid (in particular) which demonstrate less dependence in dissolution and release rate upon variation in pH in the alimentary canal.

### Summary of the Invention

The invention provides a pharmaceutical composition comprising a therapeutically effective amount of an acidic pharmacologic agent dissolved or dispersed in a polymer matrix comprising
a) from 10 weight percent to 40 weight percent of a pharmaceutically acceptable neutral, water-swellable, hydrophilic polymer, comprising a polymer selected from the group consisting of methylcellulose, hydroxymethylcellulose, hydroxyethylcellulose, hydroxypropylcellulose, carboxymethylcellulose, hydroxypropyl methylcellulose and copolymers thereof, poly(ethylene oxide) polymer, xanthum gum, and alginate;
   and
b) from 15 weight percent to 50 weight percent of a pharmaceutically acceptable acid soluble polymer which is water swellable above pH 5, which comprises a methacrylate polymer modified with a basic functional group,
   all percentages based upon the total weight of the formulation, said acidic pharmacologic agent being a compound having therapeutic activity and exhibiting a pka value less than 6.0.

The invention also provides a process for preparing a granular pharmaceutical composition suitable for pressing into tablets comprising the steps of
a) dry blending a mixture of from 5 weight percent to 50 weight percent of an acidic pharmacologic agent, said acidic pharmacologic agent being a compound having therapeutic activity and exhibiting a pka value less than 6.0, from 20 weight percent to 40 weight percent of a neutral, water-swellable hydrophilic polymer, and from 20 weight percent to 50 weight percent of an acid soluble polymer which is water-swellable at pH values above 5 to form a uniform mixture;
b) wet granulating the dry uniform mixture from step a); and
c) drying and sizing the wet granules from step b),
wherein all percentages are based upon to total weight of the granulation, wherein said neutral water-swellable hydrophilic polymer is selected from the group consisting of methylcellulose, hydroxymethylcellulose, hydroxyethylcellulose, hydroxypropylcellulose, carboxymethylcellulose, and hydroxypropyl methylcellulose and copolymers thereof, and wherein said acid-soluble polymer which is water swellable at pH values above 5 is a dialkylaminoalkyl-modified methacrylate polymer.

The formulations provide for enhanced release rate of the acidic pharmacologic agent in the stomach where the pH of gastric juices is low, and diminished release rate of the acidic pharmacologic agent at neutral or slightly alkaline pH in the intestinal tract. The result is more uniform release of the pharmacologic agent as the agent moves from the acidic environment of the stomach to the neutral or slightly alkaline environment of the upper and lower intestinal tracts. In addition, the release of the agent has less dependency upon the time the agent is resident in the acidic environment of the stomach.

In a preferred embodiment, the present invention provides a method of preparing a controlled release tablet dosage form of an acidic pharmacologic agent comprising the steps of (a) dry blending a mixture of from about 5 weight percent to about 50 weight percent of an acidic pharmacologic agent, from about 20 weight percent to about 40 weight percent of a neutral, water-swellable hydrophilic polymer, from about 20 weight percent to about 50 weight percent of an acid soluble polymer which is water-swellable at pH values above about 5 to form a uniform mixture; (b) wet granulating the dry uniform mixture from step (a); (c) drying and sizing the wet granules from step (b); and (d) compressing the blended granules of step (c) under a force ranging between (about 2000 lbf) about 8.9 x 10³ Newtons and (about 10,000 lbf) about 4:45 x 10⁴ Newtons.

### Brief Description of the Drawing Figures

In the Drawings:
FIGURE 1 is a graphical representation of data showing the percent release over time of drug at various values of pH from a control pharmaceutical formulation comprising a polymer matrix extended release formulation, but lacking any ingredient intended to moderate the pH effect.
FIGURE 2 is a graphical representation ofdata showing the percent release over time of drug at various values of pH from a formulation comprising a polymer matrix extended release formulation, and further containing anhydrous dibasic calcium phosphate.
FIGURE 3 is a graphical representation of data showing the percent release over time of drug at various values of pH from a formulation comprising a polymer matrix extended release formulation, and further containing amorphous magnesium aluminometasilicate.
FIGURE 4 is a graphical representation of data showing the release rate over time of drug at various values of pH for the preferred formulation of the present invention.
FIGURE 5 is a graphical representation of data showing the effect of *in vitro* gastric residence time on the release of valproic acid from a prior art hydroxypropyl methylcellulose matrix formulation.
FIGURE 6 is a graphical representation of data showing the effect of *in vitro* gastric residence time on the release of valproic acid from a formulation in accordance with the present invention.

### Detailed Description of the Preferred Embodiments

As used throughout this specification and the appended claims, the terms "sustained or extended release", "prolonged release", and "controlled release", as applied to drug formulations, have the meanings ascribed to them in "Remington's Pharmaceutical Sciences," 18th Ed., p.1677, Mack Pub. Co., Easton, PA (1990). Sustained or extended release drug systems include any drug delivery system which achieves the slow release of drug over an extended period of time, and include both prolonged and controlled release systems. If such a sustained release system is effective in maintaining substantially constant drug levels in the blood or target tissue, it is considered a controlled release drug delivery system. If, however, a drug delivery system is unsuccessful at achieving substantially constant blood or tissue drug levels, but nevertheless extends the duration of action of a drug over that achieved by conventional delivery, it is considered a prolonged release system.

The formulations of the present invention provide an extended or prolonged release formulation for acidic pharmacologic agents. By the term "acidic pharmacologic agent" is meant any compound having therapeutic activity and exhibiting a pKa value less than about 6.0. While not holding to one theory to the exclusion of others, it is believed that the formulations of the present invention normalize or regularize the release rate of acidic pharmacologic agents over a wide range of pH values by the interaction of the effects on release of the two polymer components of the matrix in which the pharmacologic agent is dissolved or dispersed. At low pH values in the stomach, the acid soluble polymer is believed to have the greatest effect on release of the pharmacologic agent. In this environment, where the pharmacologic agent is typically of low solubility, the acid-soluble or ionizable polymer is protonated and begins dissolving out of the formulation matrix. This aids in the release of the pharmacologic agent in the regions of the matrix where the agent is dissolved or dispersed in this component of the polymer matrix in either or both of two ways. First, the dissolution of the acid-soluble polymer component of the matrix physically aids the release of the pharmacologic agent, and second, protonation of the acid-soluble polymer by the acidic gastric juices raises the pH in the local environment of the formulation matrix to levels where the acidic pharmacologic agent is more soluble.

At the neutral or alkaline pH values encountered in the intestine, the acid-soluble polymer swells, along with the neutral water-swellable second component of the formulation polymer matrix, thus presenting a more tortuous route through which the acidic pharmacologic agent must migrate to be released from the formulation matrix. In this manner, the release rate of the pharmacologic agent is both accelerated at low pH and diminished at higher pH to provide a more uniform or regularized release rate over a wide pH range.

Moreover, since the release of the acidic pharmacologic agent in the acidic environment of the stomach and the neutral or alkaline pH of the intestine is moderated by the polymer matrix of the formulation, the overall release of the pharmacologic agent is less dependent upon the time the agent is resident in the stomach.

**Table 1**

| Typical Gastric Residence Times (GRT) of Pharmaceutical Dosage Sizes | | |
|---|---|---|
| **Dosage Size** | **State** | **GRT (min.)** |
| 1 mm | Fasted | 60-150 |
| 3 mm | Fasted | 15-240 |
| 14 mm | Fasted | 15-210 |
| 1 mm | Fed | 101 ±53 |
| 3.2 mm | Fed | 152 ±40 |
| 9 mm | Fed | 105 >600 |
| 14 mm | Fed | 180 >780 |

Table 1, reproduced from Dressman, et al., Pharm. Res., 15:1 (1998) shows the gastric residence time (GRT) of drug formulations of various dosage size in patients in both the fed and fasting states.

In a preferred embodiment, the invention provides an oral dosage form of valproic acid having diminished dependence of the release rate on pH. The term "valproic acid" is meant to encompass the compound 2-propylpentanoic acid *per se*, and salts thereof including the complex formed between one mole of 2-propylpentanoic acid and its sodium salt, commonly referred to as divalproex sodium. Divalproex sodium is disclosed in United States Patents 4,988,731 and 5,212,326 to Meade and can be represented by the following formula where m ranges from two to about six:

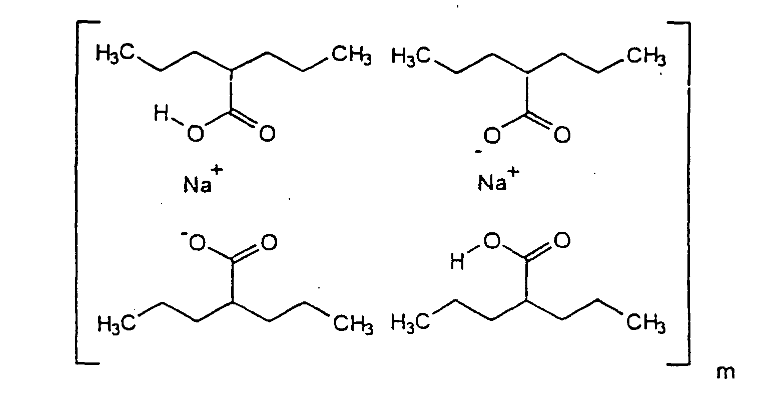

Divalproex sodium is a typical acidic pharmacologic agent, having a pKa of 4.8 and, as shown in Table 2, has a wide-ranging solubility over the pH range between pH 1.0 and pH 6.7, that is, over the range of pH difference between the stomach and the intestinal tract. It is therefore a good example of an acidic pharmacologic agent for illustrating the formulations of the present invention.

**Table 2**

| Solubility of Divalproex Sodium at Various Values of pH | |
|---|---|
| pH | Solubility (mg/mL |
| 1.0 | 1.0 |
| 4.7 | 2.0 |
| 4.84 | 3.4 |
| 5.87 | 21.0 |
| 6.17 | 36.0 |
| 6.7 | 200 |

In arriving at the present invention, various systems were contemplated to obtain a formulation which would enhance the solubility/release of an acidic pharmacologic agent at low pH and retard its release at higher pH. In this manner, the release of the agent would be more regularized as it passed from the stomach into the intestinal tract. Divalproex sodium was selected as a representative example of an acidic pharmacologic agent.

One approach involved embedding the drug in a polymer matrix along with an basic (alkaline) excipient which had the desired compressibility for tabletting, which was compatible with the other ingredients of the formulation, and which would enhance the solubility/release in the acidic environment of the stomach by raising the pH in the local environment of the matrix and by rapid leaching. In this manner, it was expected that the acidic drug would be released more quickly by the enhanced solubility/release of the excipient than from a similar polymer matrix lacking such an excipient. On the other hand, it was expected that the normally rapid dissolution/release of the acidic pharmacologic agent at the higher pH values encountered in the intestinal tract would be slowed somewhat by the need for the drug to diffuse through the polymer matrix and through the remaining undissolved excipient in the formulation which becomes insoluble in the intestine.

Using this approach, two formulations were tested. In a first formulation (Formulation 3 below), an anhydrous dibasic calcium phosphate which is soluble in acidic pH, but insoluble at neutral pH, and has superior compressibility and disintegration, was mixed, together with the drug, hydroxypropylmethyl cellulose (Methocel® grade K4MP CR, Dow Chemical), and lactose, and pressed into tablets. The anhydrous dibasic calcium phosphate used is described in United States Patent 5, 486,365 and is available as Fujicalin® from Fuji Chemical Industries, Inc.

In a second formulation (Formulation 4 below), a finely powdered amorphous magnesium aluminosilicate, also being basic and having superior compressibility and dispersion, was substituted for the dibasic calcium phosphate. This material is available as Neusilin®, also from Fuji Chemical Industries, Inc. For comparative purposes, an extended release formulation (Formulation 2) of divalproex sodium based upon a polymer matrix system, but lacking either of the two excipients described above was used as a comparative control.

As shown by the data compiled in the accompanying drawing figures, neither the approach utilized in Formulation 3 nor Formulation 4 resulted in the desired regularization of drug release over a wide range of pH, nor did the typical matrix formulation comprising a neutral water-swellable polymer as in Formulation 2. Only the preferred formulation of the invention (Formulation 1), which comprised a matrix of a neutral water-swellable polymer, and a polymer which was both acid-soluble and water swellable at higher pH produced the desired results.

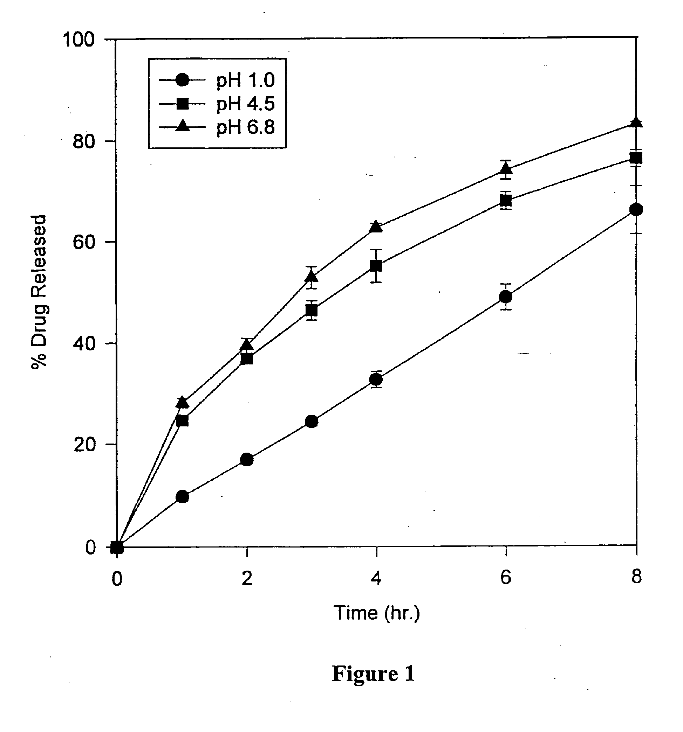

Figure 1 shows the percent release of drug over time from the control formulation (Formulation 2). Formulation 2 was a matrix extended release formulation of the active drug, but lacking any ingredient intended to moderate the pH effect. Three curves are shown, depicting the release of the drug at pH 1.0, pH 4.5 and pH 6.8. The graph clearly shows slower release of the drug at pH 1.0 than at the higher pH values as would be expected of an acidic pharmacologic agent. Moreover there is an undesirable range, after eight hours, in the total of released drug.

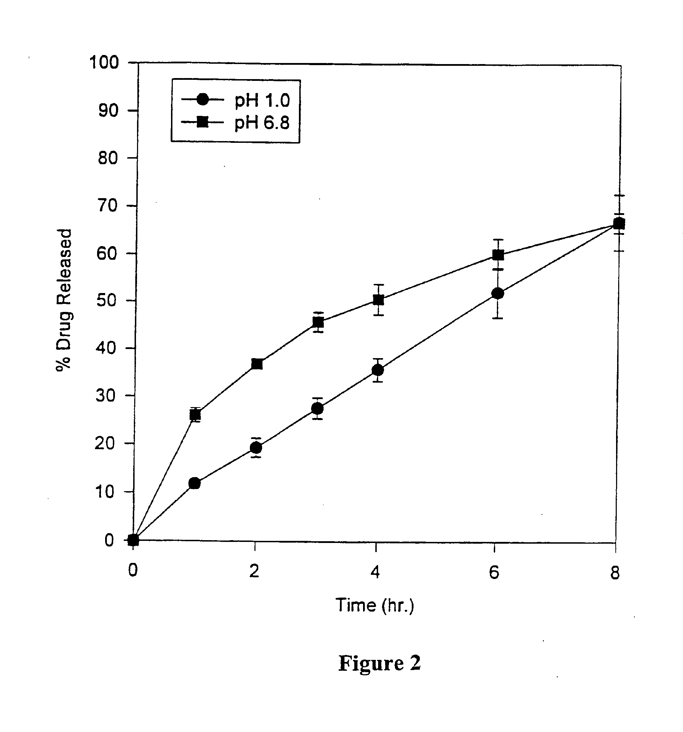

Figure 2 depicts the percent release over time of drug from the formulation containing anhydrous dibasic calcium phosphate (Formulation 3) at pH 1.0 and pH 6.8. In this instance, the total drug released after eight hours is the same at both pH values, but there is still a difference before that point in release rate of the drug at the different pH values.

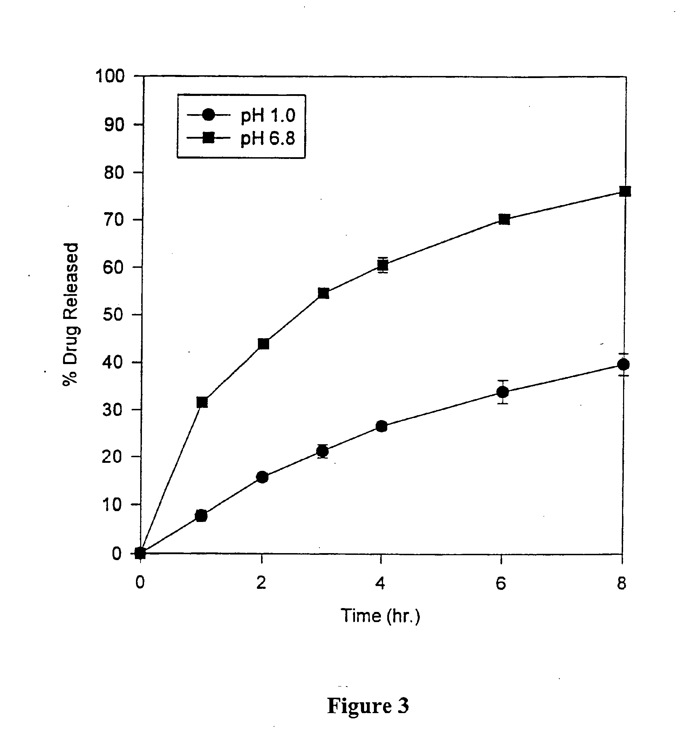

Figure 3 shows the percent release over time of drug from the formulation containing amorphous magnesium aluminometasilicate (Formulation 4) at pH 1.0 and pH 6.8. The result is clearly unacceptable as a solution to the problem of alleviating pH dependency upon drug release rate. The rates at the two values of pH vary widely, and the total amount of drug released at the lower pH after eight hours is unacceptably low.

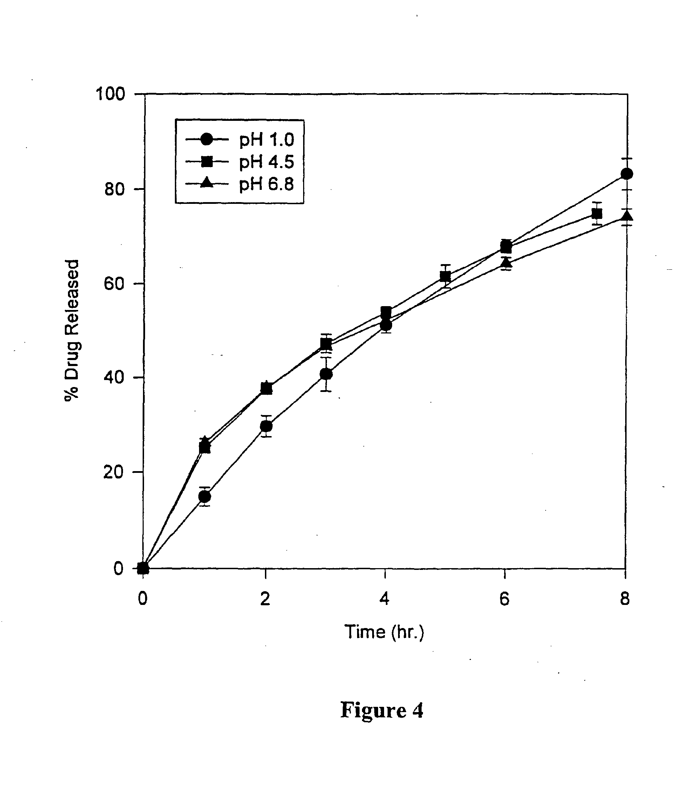

Figure 4 depicts the percent release over time of drug from the preferred formulation of the present invention (Formulation 1) at pH 1.0, 4.5 and 6.8. As can be seen by the figure, the three curves depicting percent release of the drug track one another comparatively closely.

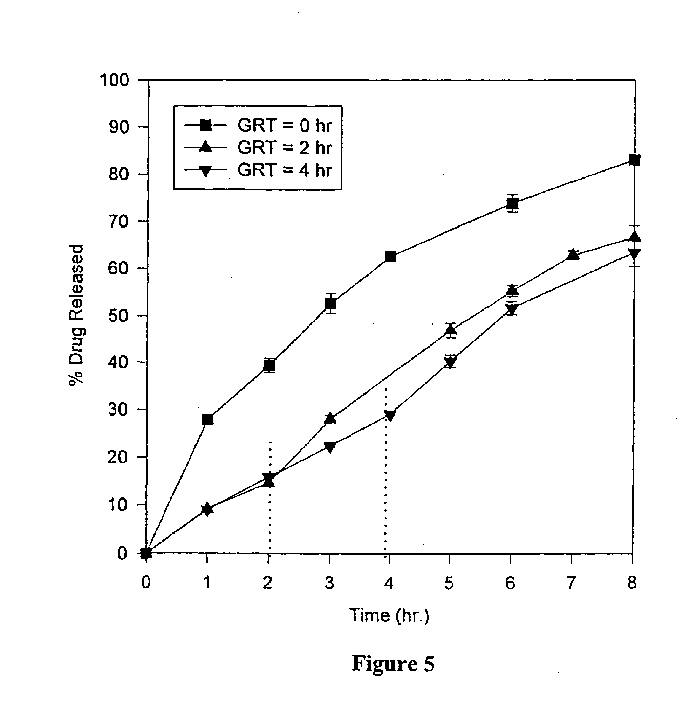

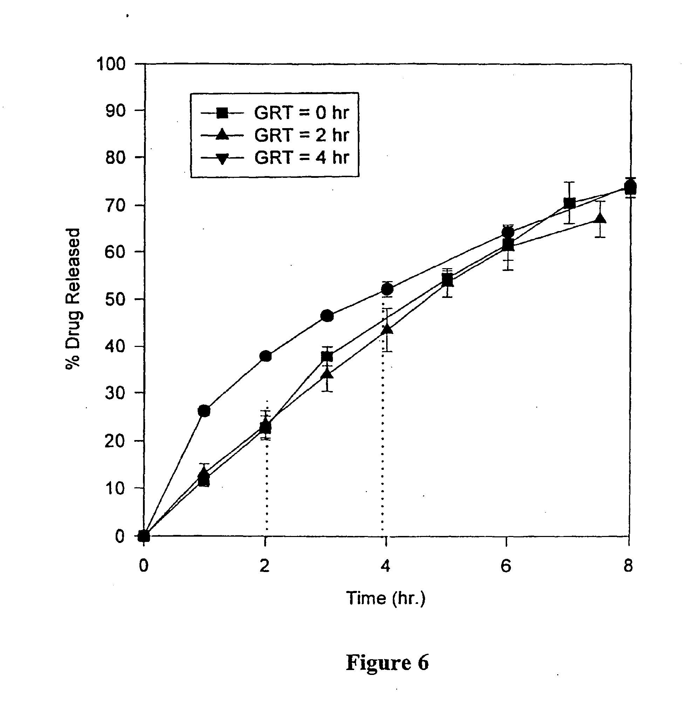

The dependence of release of valproic acid on gastric residence time from two formulations was also tested. One formulation was a typical prior art formulation in which the drug was dispersed in a hydroxypropyl methylcellulose matrix, and the other was a formulation according to the present invention in which the drug was in a matrix comprising a mixture of a neutral hydroxypropyl methylcellulose polymer and an acid-soluble dimethylaminoethyl-modified methacrylate polymer. The drug formulations were exposed to 0.1 M HCl solution *in vitro* to simulate gastric conditions and in pH 6.8 buffer (tribasic phosphate) to simulate intestinal conditions in a USP Apparatus II stirring apparatus, and the amount of drug released measured by fluorescent polarization immunoassay as described below. Zero hours gastric residence time data represent data from experiments in which the release rate from the formulations were followed from the beginning in tribasic phosphate buffer at pH 6.8. Two- and four-hour gastric residence time data are data from experiments in which the drug formulations were initially exposed for 2 or 4 hours, respectively to 0.1 M HCl and, for the remainder of the experiments to pH 6.8 tribasic buffer. The latter experiments thus simulate exposure of the drug to the acidic environment of the stomach for a pre-determined period, followed by exposure to the neutral or slightly alkaline intestinal environment for the remaining time of the experiment. The results are depicted in Figures 5 and 6.

In Figure 5, the release rate from the prior art formulation shows a noticeable increase in the release rate (slope of the curve) upon change in pH (dotted lines at 2 hours and 4 hours) as would be expected when the acidic drug "moves" from the acidic environment of the stomach to the higher pH of the intestine where it is more soluble. In addition, with the prior art formulation of Figure 5, there is a wider gap between the curves for the 2-hour and 4-hour gastric residence times. However, the data curves in Figure 6 track one another much more closely where the drug is in a formulation of the present invention. The change in release rate after 2 hours gastric residence time is considerably less, and in the case of 4 hours gastric residence time is unnoticeable. These data indicate that the formulations of the present invention demonstrate reduced dependence of release upon gastric residence time in addition to their reduced dependence upon pH.

### Examples

### Formulation 1 (Present Invention)

Four-hundred-milligram tablets containing 60 mg of divalproex sodium, 140 mg of a copolymer of a dimethylaminoethyl-modified methacrylate and neutral methacrylates of weight average molecular weight about 150,000 Daltons ("Eudragit E-100," Röhm America), 100 mg of hydroxypropylmethyl cellulose (Methocel®, grade K4MP CR, Dow Chemical), and 100 mg of lactose. This formulation represented a drug loading of 15% by weight.

Bulk drug was milled prior to use. The milled bulk drug was dry-mixed with polymer and excipients. Tablets weighing 400 mg were pressed in a Model C Carver Press tableting machine using a round die (diameter = 0.95 cm) at a compression force of about 2000 lbf (about 8.9 x 10³ Newtons).

### Formulation 2 - (Control Example) Comparative Example

Tablets were prepared by the same method, and having the same composition, as described in Formulation 1 above, but lacking the dimethylaminomethyl-modified methacrylate polymer. The drug was dispersed in a hydroxypropylmethyl cellulose (HPMC) matrix.

### Test Formulation 3 - (Comparative Example)

Tablets were prepared by the same method, and having the same composition, as described above in Formulation 1, containing 140 mg of anhydrous dicalcium phosphate ("Fujicalin," Fuji Chemical Industries, Inc.) in place of the dimethylaminomethyl-modified methacrylate polymer.

### Formulation 4 - (Comparative Example)

Tablets were prepared by the same method, and having the same composition, as described above in Formulation 1, but containing 140 mg of magnesium aluminometasilicate ("Neusilin," Fuji Chemical Industries, Inc.) in place of the dimethylaminomethyl-modified methacrylate polymer.

*In vitro* dissolution tests in aqueous solutions at various pH values were conducted with each of the formulations described above using Apparatus II and the method detailed in the *United States Pharmacopeia XXI*/*National Formulary XVI*. The stirrer paddle speed of the apparatus was 100 rpm, and the temperature of the medium was maintained at 37°C. Dissolution of each of the formulations was observed and measured at two or more of three pH values: pH 1 (0.1 M HCl), pH 4.5 (0.05 M phosphate buffer), and pH 6.8 (0.05 M tribasic phosphate buffer).

Sample aliquots of 1.5 ml were withdrawn from the stirred samples at 0, 1, 2, 3, 4, 6 and 8 hours and filtered through a 0.45 µm filter. The aliquot samples were assayed for drug by fluorescent polarization immunoassay using the TDX® analyzer (Abbott Laboratories). Upon withdrawal of each sample, an equal volume of medium was added to the test mixture to maintain constant volume. The test results are shown in Figures 1-4,

### Preferred Formulations of the Invention

Because of their superiority in controlling and regularizing the release of an acidic pharmacologic agent (e.g. divalproex sodium) over a wide pH range, compositions corresponding to Formulation 1 above comprising a neutral water-swellable polymer in combination with a polymer which is both soluble at acidic pH and water-swellable at neutral or alkaline pH, is the preferred formulation of the present invention. The formulation comprises a therapeutically effective amount of an acidic pharmacologic agent dissolved or dispersed in a polymer matrix comprising from about 10 weight percent to about 40 weight percent, preferably from about 15 weight percent to about 30 weight percent, of a pharmaceutically acceptable neutral, water-swellable, hydrophilic polymer selected from the group consisting of methylcellulose, hydroxymethylcellulose, hydroxyethylcellulose, hydroxypropylcellulose, carboxymethylcellulose, hydroxypropyl methylcellulose and copolymers thereof, poly(ethylene oxide) polymer, xanthum gum, and alginate, in combination with from about 20 weight percent to about 50 percent, preferably from about 25 weight percent to about 40 weight percent of a pharmaceutically acceptable acid soluble polymer which is water swellable above pH 5, which comprises a methacrylate polymer modified with a basic functional group, all percentage by weight based upon the total weight of the formulation. The balance of the formulation comprises pharmaceutically acceptable excipients such as diluents, binders, lubricants, glidants, disintegrating agents, and/or colorants and flavoring agents of the type generally known in the formulation arts and detailed further below.

Neutral, water-swellable, hydrophilic polymers suitable for use in the formulations of this invention include methylcellulose, hydroxymethylcellulose, hydroxyethylcellulose, hydroxypropylcellulose, carboxymethylcellulose, and hydroxypropyl methylcellulose and copolymers thereof. Neutral water-swellable modified cellulose polymers which may be used in the formulations of the present invention include, for example, Methocel®, grades E10MP CR, E4MP, K100V, K4MP, K15MP and K100MP (available from Dow Chemical); Klucel® HXF hydroxypropyl cellulose (available from Hercules, Inc.); Keltone®, grades LVCR and HVCR alginates (available from Kelco Co.), Polyox®, polyethylene oxide polymer (available from Union Carbide Co.), Keltrol®, xanthan gum (available from Kelco Co.), and Carbopol®, grades 934P, 971P, and 974P (available from B.F. Goodrich Specialty Chemicals). A preferred neutral, water-swellable polymer for formulations of the present invention is Methocel® K4MP, a cellulose ether comprising about 19-24% methoxyl substitution and about 7-12% hydroxypropyl substitution having a 2% aqueous nominal viscosity of about 4000 centipoise (4 pascal-seconds).

Polymers for use in formulations of the present invention which are acid-soluble and which swell in water at higher values of pH, are methacrylic acid/methacrylic ester copolymers which have been modified by the incorporation of a basic functional group such as an amino-, alkylamino-, dialkylamino-, or dialkylaminoalkyl-grpup. These materials are soluble at acidic pH values because of protonation at the sites of basic nitrogen atoms contained within the polymer to form ionized ammonium groups. These materials are also thus designated as "acid ionizable" or "salt-forming" methacrylic-acid/methacrylic ester copolymers. Acid-soluble modified methacrylate polymers which swell in water at neutral or alkaline pH values which may be used in formulations of the present invention include Eudragit® E100 which is a cationic copolymer based on dimethylaminoethyl methacrylate and neutral methacrylates, having a molecular weight of about 150,000 Daltons, (available from Röhm America, Inc.).

### Excipients

In addition to the active pharmacologic agent and polymer matrix, pre-compressed or compressed formulations of the present invention may contain additives or excipients which act in one or more capacities as diluents, binders, lubricants, glidants, disintegrating agents, colorants or flavoring agents.

In those situations where the amount of active pharmacologic agent in a tablet is small, one or more inert diluents is added to increase the bulk of the tablet. Diluents used for this purpose include dicalcium phosphate, calcium sulfate, dextrose, amylose, lactose, microcrystalline cellulose, kaolin, mannitol, sodium chloride, dry starch and powdered sugar.

Binders impart cohesiveness to the tablet formulation, insuring that the tablet remains intact after compression, as well as improving the free-flowing characteristics and desired hardness of pre-tabletting granulations. Materials commonly used as binders include starch, gelatin, and sugars such as sucrose, glucose, dextrose, molasses and lactose. Natural and synthetic gums such as acacia, sodium alginate, extract of Irish moss, panwar gum, ghatti gum, carboxymethylcellulose, methyl cellulose, and polyvinylpyrrolidone may also be used as binders.

Lubricants serve a number of functions in tablet formulation including the prevention of adhesion of the pressed tablet to the surface of dies and punches, facilitation of release of the tablet from the die, and improving the flow rate of pre-tabletting granulations. Commonly used lubricants include talc, magnesium stearate, calcium stearate, stearic acid, hydrogenated vegetable oils and poly(ethylene glycol). The quantity of lubricant used in the tablet formulation may varying between a low of about 0.1 weight percent to as high as about 5 weight percent.

Glidants improve the flow characteristics of dry powdered mixtures. Colloidal silicon dioxide is most commonly used as a glidant, although asbestos-free talc is also sometimes used. The glidant, when used, typically constitutes about 1 weight percent or less of the formulation.

Disintegrating agents, either as single substances or as mixtures, are added to tablet formulations to facilitate the break-up or disintegration of the tablet after administration. Com and potato starch, which have been well dried and powdered, are the most commonly used tablet disintegrating agents. The amount of starch added to the formulation varies, depending upon the rate of disintegration desired, and ranges between about 5 weight percent and about 10 to 15 weight percent. Swelling disintegrating agents such as croscarmelose, crospovidone, and sodium starch glycolate represent, respectively, examples of cross-linked cellulose, cross-linked polymer, and cross-linked starch agents which may also be used. These materials are typically used in amounts ranging between about 2 and 4 weight percent in the tablet formulation.

Coloring agents may be added to the tablet formulation or to a polymeric material used as a tablet coating. Suitable coloring agents include those approved for use by the United States Food and Drug Administration (FDA) and are well known to those of the formulation arts.

### Tabletting Processes

Tablets are generally prepared by one of three methods well known to those skilled in the art: wet granulation, dry granulation, or direct compression. Any of the three methods may be used to formulate tablets in accordance with the present invention.

## Claims

1. A pharmaceutical composition comprising a therapeutically effective amount of an acidic pharmacologic agent dissolved or dispersed in a polymer matrix comprising
a) from 10 weight percent to 40 weight percent of a pharmaceutically acceptable neutral, water-swellable, hydrophilic polymer, comprising a polymer selected from the group consisting of methylcellulose, hydroxymethylcellulose, hydroxyethylcellulose, hydroxypropylcellulose, carboxymethylcellulose, hydroxypropyl methylcellulose and copolymers thereof, poly(ethylene oxide) polymer, xanthum gum, and alginate;
and
b) from 15 weight percent to 50 weight percent of a pharmaceutically acceptable acid soluble polymer which is water swellable above pH 5, which comprises a methacrylate polymer modified with a basic functional group,
all percentages based upon the total weight of the formulation, said acidic pharmacologic agent being a compound having therapeutic activity and exhibiting a pka value less than 6,0.

2. The pharmaceutical composition according to Claim 1 wherein said polymer matrix comprises from 20 weight percent to 30 weight percent of said pharmaceutically acceptable neutral, water-swellable, hydrophilic polymer.

3. The pharmaceutical composition according to Claim 1 wherein said polymer matrix comprises from 20 weight percent to 40 weight percent of said pharmaceutically acceptable acid-soluble polymer which is water swellable above pH5.

4. The pharmaceutical composition of Claim 1 wherein said acid-soluble polymer which is water-swellable above pH 5 comprises a dialkylaminoalkyl- modified methacrylate polymer.

5. A pharmaceutical composition according to claim 1, wherein the pharmaceutically acceptable neutral, water-swellable, hydrophilic polymer is pharmaceutically acceptable neutral, water-swellable, cellulosic polymer, and wherein the pharmaceutically acceptable acid soluble polymer is an acid soluble polymer which comprises a dialkylaminoalkyl-modified methacrylate polymer.

6. The pharmaceutical composition of claim 1 wherein the acidic pharmacologic agent is divalproex sodium.

7. The pharmaceutical composition of Claim 5 wherein said neutral, water-swellable, hydrophilic polymer comprises a polymer selected from the group consisting of methylcellulose, hydroxymethylcellulose, hydroxyethylcellulose, hydroxypropylcellulose, carboxymethylcellulose, and hydroxypropyl methylcellulose and copolymers thereof.

8. The pharmaceutical composition of claim 5 wherein said acid-soluble dialkylaminoalkyl-modified methacrylate polymer is a dimethylaminoethyl-modified methacrylate polymer.

9. The pharmaceutical composition of claim 5 wherein said acidic pharmacologic agent is divalproex sodium.

10. A pharmaceutical composition according to claim 1 in the form of a dosage form, comprising a therapeutically effective amount of divalproex sodium dissolved or dispersed in a matrix comprising from 10 weight percent to 40 weight percent of a neutral water-swellable cellulosic polymer and from 20 weight percent to 50 weight percent of an acid-soluble dialkylamino-modified methacrylate polymer which is water-swellable at pH values above 5, all percentages based upon the total weight of the dosage form.

11. A pharmaceutical composition according to claim 1 in the form of a dry granular composition suitable for compressing into a tablet dosage form, comprising particles containing an acidic pharmacologic agent dissolved or dispersed in a matrix comprising from 10 weight percent to 40 weight percent of a neutral, water swellable hydrophilic polymer and from 20 weight percent 50 weight percent of an acid soluble polymer which is water swellable at pH values above 5, which comprises a dialkylaminoalkyl-modified methacrylate polymer, at weight percentages based upon the total weight of the granular composition, wherein said neutral water-swellable hydrophilic polymer is selected from the group consisting of methylcellulose, hydroxymethylcellulose, hydroxyethylcellulose, hydroxypropylcellulose, carboxymethylcellulose, and hydroxypropyl methylcellulose and copolymers thereof, said acidic pharmacologic agent being a compound having therapeutic activity and exhibiting a pka value less than 6.0.

12. The granular composition of claim 11 wherein said matrix comprises from 20 weight percent to 30 weight percent of said neutral water-swellablehydrophilic polymer.

13. The granular composition of claim 11 wherein said matrix comprises from 20 weight percent to 40 weight percent of said acid soluble polymer which is water swellable at pH values above 5.

14. The granular composition of claim 11 wherein said acidic pharmacologic agent is divalproex sodium.

15. A process for preparing a granular pharmaceutical composition suitable for pressing into tablets comprising the steps of
a) dry blending a mixture of from 5 weight percent to 50 weight percent of an acidic pharmacologic agent, said acidic pharmacologic agent being a compound having therapeutic activity and exhibiting a pka value less than 6.0, from 20 weight percent to 40 weight percent of a neutral, water-swellable hydrophilic polymer, and from 20 weight percent to 50 weight percent of an acid soluble polymer which is water-swellable at pH values above 5 to form a uniform mixture;
b) wet granulating the dry uniform mixture from step a); and
c) drying and sizing the wet granules from step b),
wherein all percentages are based upon to total weight of the granulation, wherein said neutral water-swellable hydrophilic polymer is selected from the group consisting of methylcellulose, hydroxymethylcellulose, hydroxyethylcellulose, hydroxypropylcellulose, carboxymethylcellulose, and hydroxypropyl methylcellulose and copolymers thereof, and wherein said acid-soluble polymer which is water swellable at pH values above 5 is a dialkylaminoalkyl-modified methacrylate polymer.

16. The process according to claim 15, wherein said neutral, waster-swellable hydrophilic polymer comprises hydroxypropyl methyl cellulose.

17. A process according to claim 15 wherein said acidic pharmacologic agent is divalproex sodium.

18. A process according to claim 15 comprising the steps of
a) dry blending a mixture of from 5 weight percent to 50 weight percent of an acidic therapeutic agent said acidic pharmacologic agent being a compound having therapeutic activity and exhibiting a pka value less than 6.0, from 20 weight percent to 40 weight percent of a neutral, water-swellable hydrophilic polymer, from 20 weight percent to 50 weight percent of an acid soluble polymer which is water-swellable at pH values above 5 to form a uniform mixture;
b) wet granulating the dry uniform mixture from step a);
c) drying and sizing the wet granules from step b); and
d) compressing the blended granules of step c) under a force ranging between (2000 lbf) 8.9 x 10³ Newtons and (10,000 lbf) 4.45 x 10⁴ Newtons, wherein said neutral water-swellable hydrophilic polymer is selected from the group consisting of methylcellulose, hydroxymethylcellulose, hydroxyethylcellulose, hydroxypropylcellulose, carboxymethylcellulose, and hydroxypropyl methylcellulose and copolymers thereof, and wherein said acid-soluble polymer which is water swellable at pH values above 5 is a dialkylaminoalkyl-modified methacrylate polymer to prepare a controlled release tablet dosage form.

19. The process according to claim 18, wherein said neutral, water-swellable hydrophilic polymer comprises hydroxypropyl methyl cellulose.

20. A process according to Claim 18 wherein said acidic pharmacologic agent is divalproex sodium.

## Patentansprüche

1. Pharmazeutische Zusammensetzung, umfassend eine therapeutisch wirksame Menge eines sauren pharmakologischen Wirkstoffs, aufgelöst oder dispergiert in einer Polymermatrix, die folgendes umfaßt:
a) von 10 Gewichtsprozent bis 40 Gewichtsprozent eines pharmazeutisch verträglichen neutralen, in Wasser quellbaren, hydrophilen Polymers, umfassend ein Polymer gewählt aus der Gruppe bestehend aus Methylzellulose, Hydroxymethylzellulose, Hydroxyethylzellulose, Hydroxypropylzellulose, Carboxymethylzellulose, Hydroxypropylmethylzellulose und Copolymere davon, Poly(ethylenoxid)polymer, Xanthangummi und Alginat;
und
b) von 15 Gewichtsprozent bis 50 Gewichtsprozent eines pharmazeutisch verträglichen säurelöslichen Polymers, welches oberhalb pH 5 in Wasser quellbar ist, welches ein Methacrylatpolymer umfaßt, modifiziert mit einer basischen funktionellen Gruppe, wobei alle Prozentsätze auf dem Gesamtgewicht der Formulierung basieren, wobei der saure pharmakologische Wirkstoff eine Verbindung mit therapeutischer Aktivität ist, die einen pka-Wert von weniger als 6,0 hat.

2. Pharmazeutische Zusammensetzung gemäß Anspruch 1, worin die Polymermatrix von 20 Gewichtsprozent bis 30 Gewichtsprozent des pharmazeutisch verträglichen neutralen, in Wasser quellbaren, hydrophilen Polymers umfaßt.

3. Pharmazeutische Zusammensetzung gemäß Anspruch 1, worin die Polymermatrix von 20 Gewichtsprozent bis 40 Gewichtsprozent des pharmazeutisch verträglichen säurelöslichen Polymers umfaßt, welches oberhalb pH 5 in Wasser quellbar ist.

4. Pharmazeutische Zusammensetzung gemäß Anspruch 1, worin das säurelösliche Polymer, welches oberhalb pH 5 in Wasser quellbar ist, ein Dialkylaminoalkyl-modifiziertes Methacrylatpolymer umfaßt.

5. Pharmazeutische Zusammensetzung gemäß Anspruch 1, worin das pharmazeutisch verträgliche neutrale, in Wasser quellbare, hydrophile Polymer ein pharmazeutisch verträgliches neutrales, in Wasser quellbares Zellulosepolymer ist und worin das pharmazeutisch verträgliche säurelösliche Polymer ein säurelösliches Polymer ist, welches ein Dialkylaminoalkyl-modifiziertes Methacrylatpolymer umfaßt.

6. Pharmazeutische Zusammensetzung gemäß Anspruch 1, worin der saure pharmakologische Wirkstoff Divalproexnatrium ist.

7. Pharmazeutische Zusammensetzung gemäß Anspruch 5, worin das neutrale, in Wasser quellbare, hydrophile Polymer ein Polymer umfaßt, das gewählt ist aus der Gruppe bestehend aus Methylzellulose, Hydroxymethylzellulose, Hydroxyethylzellulose, Hydroxypropylzellulose, Carboxymethylzellulose und Hydroxypropylmethylzellulose und Copolymeren davon.

8. Pharmazeutische Zusammensetzung gemäß Anspruch 5, worin das säurelösliche Dialkylaminoalkyl-modifizierte Methacrylatpolymer ein Dimethylaminoethyl-modifiziertes Methacrylatpolymer ist.

9. Pharmazeutische Zusammensetzung gemäß Anspruch 5, worin der saure pharmakologische Wirkstoff Divalproexnatrium ist.

10. Pharmazeutische Zusammensetzung gemäß Anspruch 1 in Form einer Dosierform, umfassend eine therapeutisch wirksame Menge von Divalproexnatrium, aufgelöst oder dispergiert in einer Matrix, die von 10 Gewichtsprozent bis 40 Gewichtsprozent eines neutralen, in Wasser quellbaren Zellulosepolymers und von 20 Gewichtsprozent bis 50 Gewichtsprozent eines säurelöslichen Dialkylamino-modifizierten Methacrylatpolymers, welches bei pH-Werten oberhalb von 5 in Wasser quellbar ist, umfaßt, wobei alle Prozentsätze auf dem Gesamtgewicht der Dosierform basieren.

11. Pharmazeutische Zusammensetzung gemäß Anspruch 1 in Form einer trockenen granulären Zusammensetzung, die zur Kompression in eine Tabletten-Dosierform geeignet ist, umfassend Partikel, die einen sauren pharmakologischen Wirkstoff enthalten, aufgelöst oder dispergiert in einer Matrix, die von 10 Gewichtsprozent bis 40 Gewichtsprozent eines neutralen, in Wasser quellbaren, hydrophilen Polymers und von 20 Gewichtsprozent bis 50 Gewichtsprozent eines säurelöslichen Polymers umfaßt, welches bei pH-Werten oberhalb von 5 in Wasser quellbar ist, welches ein Dialkylaminoalkyl-modifiziertes Methacrylatpolymer umfaßt, wobei alle Gewichtsprozentsätze auf dem Gesamtgewicht der granulären Zusammensetzung basieren, worin das neutrale in Wasser quellbare, hydrophile Polymer gewählt ist aus der Gruppe bestehend aus Methylzellulose, Hydroxymethylzellulose, Hydroxyethylzellulose, Hydroxypropylzellulose, Carboxymethylzellulose und Hydroxypropylmethylzellulose und Copolymeren davon, wobei der saure pharmakologische Wirkstoff eine Verbindung ist, die therapeutische Wirksamkeit besitzt und einen pka-Wert von weniger als 6,0 aufweist.

12. Granuläre Zusammensetzung gemäß Anspruch 11, worin die Matrix von 20 Gewichtsprozent bis 30 Gewichtsprozent des neutralen, in Wasser quellbaren, hydrophilen Polymers umfaßt.

13. Granuläre Zusammensetzung gemäß Anspruch 11, worin die Matrix von 20 Gewichtsprozent bis 40 Gewichtsprozent des säurelöslichen Polymers umfaßt, welches bei pH-Werten oberhalb von 5 in Wasser quellbar ist.

14. Granuläre Zusammensetzung gemäß Anspruch 11, worin der saure pharmakologische Wirkstoff Divalproexnatrium ist.

15. Verfahren zur Herstellung einer granulären pharmazeutischen Zusammensetzung, die geeignet ist zur Kompression in Tabletten, das die folgenden Schritte umfaßt:
a) Trockenmischen einer Mischung aus von 5 Gewichtsprozent bis 50 Gewichtsprozent eines sauren pharmakologischen Wirkstoffs, wobei der saure pharmakologische Wirkstoff eine Verbindung ist, die therapeutische Aktivität besitzt und einen pka-Wert von weniger als 6,0 aufweist, von 20 Gewichtsprozent bis 40 Gewichtsprozent eines neutralen, in Wasser quellbaren, hydrophilen Polymers, und von 20 Gewichtsprozent bis 50 Gewichtsprozent eines säurelöslichen Polymers, welches bei pH-Werten oberhalb von 5 in Wasser quellbar ist, um eine gleichförmige Mischung zu bilden;
b) nasses Granulieren der gleichförmigen Trockenmischung aus Schritt a) und
c) Trocknen und Sieben der nassen Granulate aus Schritt b),
worin alle Prozentsätze auf dem Gesamtgewicht der Granulation basieren, worin das neutrale in Wasser quellbare, hydrophile Polymer gewählt ist aus der Gruppe bestehend aus Methylzellulose, Hydroxymethylzellulose, Hydroxyethylzellulose, Hydroxypropylzellulose, Carboxymethylzellulose und Hydroxypropylmethylzellulose und Copolymeren davon, und worin das säurelösliche Polymer, welches bei pH-Werten oberhalb von 5 in Wasser quellbar ist, ein Dialkylaminoalkyl-modifiziertes Methacrylatpolymer ist.

16. Verfahren gemäß Anspruch 15, worin das neutrale, in Wasser quellbare, hydrophile Polymer Hydroxypropylmethylzellulose umfaßt.

17. Verfahren gemäß Anspruch 15, worin der saure pharmakologische Wirkstoff Divalproexnatrium ist.

18. Verfahren gemäß Anspruch 15, das die folgenden Schritte umfaßt:
a) Trockenmischen einer Mischung aus von 5 Gewichtsprozent bis 50 Gewichtsprozent eines sauren therapeutischen Wirkstoffs,
wobei der saure pharmakologische Wirkstoff eine Verbindung ist, die therapeutische Wirksamkeit besitzt und eine pka-Wert von weniger als 6,0 aufweist, von 20 Gewichtsprozent bis 40 Gewichtsprozent eines neutralen, in Wasser quellbaren, hydrophilen Polymers, von 20 Gewichtsprozent bis 50 Gewichtsprozent eines säurelöslichen Polymers, welches bei pH-Werten oberhalb von 5 in Wasser quellbar ist, um eine gleichförmige Mischung zu bilden;
b) nasses Granulieren der gleichförmigen Trockenmischung von Schritt a);
c) Trocknen und Sieben der nassen Granulate von Schritt b) und
d) Komprimieren der gemischten Granulate von Schritt c) unter einer Kraft im Bereich zwischen (2000 lbf) 8,9 x 10³ Newton und (10.000 lbf) 4,45 x 10⁴ Newton, worin das neutrale, in Wasser quellbare, hydrophile Polymer gewählt ist aus der Gruppe bestehend aus Methylzellulose, Hydroxymethylzellulose, Hydroxyethylzellulose, Hydroxypropylzellulose, Carboxymethylzellulose und Hydroxypropylmethylzellulose und Copolymeren davon, und worin das säurelösliche Polymer, welches bei pH-Werten oberhalb von 5 in Wasser quellbar ist, ein Dialkylaminoalkyl-modifiziertes Methacrylatpolymer ist,
um eine Tabletten-Dosierform mit kontrollierter Freisetzung herzustellen.

19. Verfahren gemäß Anspruch 18, worin das neutrale, in Wasser quellbare, hydrophile Polymer Hydroxypropylmethylzellulose umfaßt.

20. Verfahren gemäß Anspruch 18, worin der saure pharmakologische Wirkstoff Divalproexnatrium ist.

## Revendications

1. Composition pharmaceutique comprenant une quantité thérapeutiquement efficace d'un agent pharmacologique acide dissous ou dispersé dans une matrice polymère comprenant
a) de 10 % en masse à 40 % en masse d'un polymère hydrophile gonflable à l'eau neutre pharmaceutiquement acceptable comprenant un polymère choisi dans le groupe consistant en la méthylcellulose, l'hydroxyméthylcellulose, l'hydroxyéthylcellulose, l'hydroxypropylcellulose, la carboxyméthylcellulose, l'hydroxypropylméthylcellulose et leurs copolymères, un polymère de poly(oxyde d'éthylène), la gomme de xanthane et un alginate ;
et
b) de 15 % en masse à 50 % en masse d'un polymère soluble dans les acides pharmaceutiquement acceptable qui est gonflable à l'eau au-dessus de pH 5, qui comprend un polymère de méthacrylate modifié avec un groupe fonctionnel basique,
tous les pourcentages basés sur la masse totale de la formulation, ledit agent pharmacologique acide étant un composé ayant une activité thérapeutique et présentant un pka inférieur à 6,0.

2. Composition pharmaceutique selon la revendication 1 où ladite matrice polymère comprend de 20 % en masse à 30 % en masse dudit polymère hydrophile gonflable à l'eau neutre pharmaceutiquement acceptable.

3. Composition pharmaceutique selon la revendication 1 où ladite matrice polymère comprend de 20 % en masse à 40 % en masse dudit polymère soluble dans les acides pharmaceutiquement acceptables qui est gonflable à l'eau au-dessus de pH 5.

4. Composition pharmaceutique selon la revendication 1 où ledit polymère soluble dans les acides qui est gonflable à l'eau au-dessus de pH 5 comprend un polymère de méthacrylate dialkylaminoalkyl-modifié.

5. Composition pharmaceutique selon la revendication 1 où le polymère hydrophile gonflable à l'eau neutre pharmaceutiquement acceptable est un polymère cellulosique gonflable à l'eau neutre pharmaceutiquement acceptable et où le polymère soluble dans les acides pharmaceutiquement acceptable est un polymère soluble dans les acides qui comprend un polymère de méthacrylate dialkylaminoalkyl-modifié.

6. Composition pharmaceutique selon la revendication 1 où l'agent pharmacologique acide est le divalproex sodium.

7. Composition pharmaceutique selon la revendication 5 où ledit polymère hydrophile gonflable à l'eau neutre comprend un polymère choisi dans le groupe consistant en la méthylcellulose, l'hydroxyméthylcellulose, l'hydroxyéthylcellulose, l'hydropropylcellulose, la carboxymétylcellulose et l'hydroxypropylméthycellulose et leurs copolymères.

8. Composition pharmaceutique selon la revendication 5 où ledit polymère de méthacrylate dialkylaminoalkyl-modifié soluble dans les acides est un polymère de méthacrylate diméthylaminoéthyl-modifié.

9. Composition pharmaceutique selon la revendication 5 où ledit agent pharmacologique acide est le divalproex sodium.

10. Composition pharmaceutique selon la revendication 1 sous forme d'une forme galénique comprenant une quantité thérapeutiquement efficace de divalproex sodium dissous ou dispersé dans une matrice comprenant de 10 % en masse à 40 % en masse d'un polymère cellulosique gonflable à l'eau neutre et de 20 % en masse à 50 % en masse d'un polymère de méthacrylate dialkylamino-modifié soluble dans les acides qui est gonflable à l'eau à des pH supérieurs à 5, tous les pourcentages basés sur la masse totale de la forme galénique.

11. Composition pharmaceutique selon la revendication 1 sous forme d'une composition granulaire sèche appropriée pour la compression en une forme galénique de type comprimé, comprenant des particules contenant un agent pharmacologique acide dissous ou dispersé dans une matrice comprenant de 10 % en masse à 40 % en masse d'un polymère hydrophile gonflable à l'eau neutre et de 20 % en masse à 50 % en masse d'un polymère soluble dans les acides qui est gonflable à l'eau à des pH supérieurs à 5, qui comprend un polymère de méthacrylate dialkylaminoalkyl-modifié, tous les pourcentages en masse basés sur la masse totale de la composition granulaire, où ledit polymère hydrophile gonflable à l'eau neutre est choisi dans le groupe consistant en la méthylcellulose, l'hydroxyméthylcellulose, l'hydroxyéthylcellulose, l'hydroxypropylcellulose, la carboxyméthylcellulose et l'hydroxypropylméthylcellulose et leurs copolymères, ledit agent pharmacologique acide étant un composé ayant une activité thérapeutique et présentant un pka inférieur à 6,0.

12. Composition granulaire selon la revendication 11 où ladite matrice comprend de 20 % en masse à 30 % en masse dudit polymère hydrophile gonflable à l'eau neutre.

13. Composition granulaire selon la revendication 11 où ladite matrice comprend de 20 % en masse à 40 % en masse dudit polymère soluble dans les acides qui est gonflable à l'eau à des pH supérieurs à 5.

14. Composition granulaire selon la revendication 11 où ledit agent pharmacologique acide est le divalproex sodium.

15. Procédé pour préparer une composition pharmaceutique granulaire appropriée pour la compression en comprimés comprenant les étapes de :
a) mélange à sec d'un mélange de 5 % en masse à 50 % en masse d'un agent pharmacologique acide, ledit agent pharmacologique acide étant un composé ayant une activité thérapeutique et présentant un pka inférieur à 6,0, de 20 % en masse à 40 % en masse d'un polymère hydrophile gonflable à l'eau neutre et de 20 % en masse à 50 % en masse d'un polymère soluble dans les acides qui est gonflable à l'eau à des pH supérieurs à 5 pour former un mélange uniforme ;
b) granulation humide du mélange uniforme sec provenant de l'étape a) ; et
c) séchage et calibrage des granulés humides provenant de l'étape b),
où tous les pourcentages sont basés sur la masse totale des granulés, où ledit polymère hydrophile gonflable à l'eau neutre est choisi dans le groupe consistant en la méthylcellulose, l'hydroxyméthylcellulose, l'hydroxyéthylcellulose, l'hydroxypropylcellulose, la carboxyméthylcellulose et l'hydroxypropylméthylcellulose et leurs copolymères, et où ledit polymère soluble dans les acides qui est gonflable à l'eau à des pH supérieurs à 5 est un polymère de méthacrylate dialkylaminoalkyl-modifié.

16. Procédé selon la revendication 15 où ledit polymère hydrophile gonflable à l'eau neutre comprend de l'hydroxypropylméthylcellulose.

17. Procédé selon la revendication 15 où ledit agent pharmacologique acide est le divalproex sodium.

18. Procédé selon la revendication 15 comprenant les étapes de :
a) mélange à sec d'un mélange de 5 % en masse à 50 % en masse d'un agent thérapeutique acide, ledit agent pharmacologique acide étant un composé ayant une activité thérapeutique et présentant un pka inférieur à 6,0, de 20 % en masse à 40 % en masse d'un polymère hydrophile gonflable à l'eau neutre, de 20 % en masse à 50 % en masse d'un polymère soluble dans les acides qui est gonflable à l'eau à des pH supérieurs à 5 pour former un mélange uniforme ;
b) granulation humide du mélange uniforme sec provenant de l'étape a) ;
c) séchage et calibrage des granulés humides provenant de l'étape b) ; et
d) compression des granulés mélangés de l'étape c) sous une force allant de 8,9 x 10³ Newtons (2000 Ibf) à 4,45 x 10⁴ Newtons (10 000 Ibf), où ledit polymère hydrophile gonflable à l'eau neutre est choisi dans le groupe consistant en la méthylcellulose, l'hydroxyméthylcellulose, l'hydroxyéthylcellulose, l'hydroxypropylcellulose, la carboxyméthylcellulose et l'hydroxypropylméthylcellulose et leurs copolymères, et où ledit polymère soluble dans les acides qui est gonflable à l'eau à des pH supérieurs à 5 est un polymère de méthacrylate dialkylaminoalkyl-modifié,
pour préparer une forme galénique de type comprimé à libération contrôlée.

19. Procédé selon la revendication 18 où ledit polymère hydrophile gonflable à l'eau neutre comprend de l'hydroxypropylméthylcellulose.

20. Procédé selon la revendication 18 où ledit agent pharmacologique acide est le divalproex sodium.
